# EUROPEAN PATENT APPLICATION

(11) **EP 1 709 903 A1**
(43) Date of publication of application: **11.10.2006**
(21) Application number: 05447077.8
(22) Date of filing: 04.04.2005
(51) Int. Cl.: A61B 5/103

(54) **Portable wireless electronic system for the diagnosis, the monitoring and/or the therapy of dyskinesia**

(71) Applicant: UNIVERSITE LIBRE DE BRUXELLES, 1050 Brussels (BE); Instituto de Automatica Industrial, 28500 Arganda del Rey, Madrid (ES); Asociacion Instituto de Biomecanica de Valencia, 46022 Valencia (ES)
(72) Inventor: Manto, Mario, 6000 Charleroi (BE); Pons, Jose, 28007 Madrid (ES); Rocon, Eduardo, 28009 Madrid (ES); Belda, Juan Manuel, 46022 Valencia (ES); Comin, Mario, 46117 Betera (Valencia) (ES); Barbera, Ricard, 46970 Alaquàs (Valencia) (ES)
(74) Representative: Van Malderen, Joëlle

(57) **Abstract**

The present invention is related to an electronic system for the diagnosis, the monitoring and/or the treatment of dyskinesia comprising :
- a measuring unit (1) with sensors to be applied upon a human body patient,
- a Master Unit (2),
- a video recorder (9),
- a software platform (8),
- a memory device (6),
- and a drug delivery device (7) to be applied upon the human body patient.

## Description

### Field of the invention

The present invention is related to a portable wireless electronic system for the diagnosis, the monitoring and/or the therapy of dyskinesia, especially human dyskinesia.

### Background of the invention

Human dyskinesia constitutes an abnormal movement, which interferes heavily with the activities of daily living. This irregular and uncontrollable movement affects a different part of the body.

Burkhard et al. (Mov. Disord. 1999, vol. 14, p. 754-763) describes three types of dyskinesia : choreic, dystonic and mixed dyskinesia which may have various origins.

1) Parkinson disease : dyskinesia appears among 30% of the patients receiving levodopa after five years of treatment (Derkinderen et al. Rev. Neurol. Paris 2002, vol. 158, p. 92-101 and Schrag et al. Brain 2000, vol. 123, p. 2297 - 2305) and among more than 50 % of the patients after ten years of treatment (Poewe et al. Movement disorders, Watts and Koller Edition, Mac Graw Hill, 1997, p. 2001-2019).

Various risk factors have been identified : the early development of Parkinson disease, a disease having severe effects or the administration of high level of levodopa to the patient.

Furthermore, survival curves show that Parkinson disease having started in a patient before, or at 50 years, combined with an initial dose of lévodopa higher than 600 mg/day is associated with a high risk of dyskinesia (p < 0.05) (Grandas et al. J. Neurol. 1999, vol. 246, p. 1127 - 1133). For these patients, dyskinesia presents an important biphasic character, which means that such dyskinesia appears at the begin of the administration and in the end of the administration, or can be present during the peak dose administration (peak dose dyskinesia).

These dyskinesia are preferably present upon the body side firstly affected when early symptoms are discovered (Grandas et al. 1999).

During the evolution of the Parkinson disease, the distinction between the various type of dyskinesia is more difficult, because an overlap exists with other detected abnormal movements (myoclonus, akathisia and pseudoakathisia) (Derkinderen et al. 2002, Havaki-Kontaxahi et al. Eur. Neuropsychopharmacol. 2000, vol. 10, p. 333-336).

Pulsate stimulation of Dopaminergic nerves can also generated dyskinesia (Jenner Neurology 2004, vol. 62, p. 47-55).

Furthermore, preliminary studies upon primates show that the addition of small doses of lévodopa to a COMT (cathechol-o-methyl transferase) inhibitor (such as entacapone) or to a dopaminergic agonist can avoid dyskinesia in toxic models of Parkinsonism (Jenner 2004).

2. Multi-systemic atrophy : dyskinesia appears earlier than with Parkinson disease.

3. Schizophrenia : dyskinesia appears spontaneously or following the administration of neuroleptics (McCreadie et al. Br. J. Psychiatry 2002, vol. 181, p. 135-137).

The natural evolution is not well known because patients can also suffer from akathisis or pseudoakathisis. Pseudoakathisis is present in about 5% of schizophrenic patients (Havaki-Kontaxaki et al. 2000).

Natural evolution of this pseudo akathisis is not well known. Among patients receiving a poly-therapy treatment, dyskinesias are often detected after complex clinical studies and are usually not well characterized from these clinical analyses.

Furthermore, classical neuroleptics (such as Haloperidol) are usually associated with a higher incidence of diskinesia than recently developed anti-psychotic drugs (such as Risperidone). However, further studies are required upon these new compounds.

4. CO Intoxication : dyskinesia usually disappears after 8 weeks after the diagnosis (Choi et al. Eur. Neurol. 1999, vol. 42, p. 141-144).

More rarely, Parkinson disease can also be associated with a dysfunction of a neuro-muscular junction, coupled with clinical lesions of the central neuronal system and the peripheral neuronal system (Ueno et al. Neurology 1987, vol. 37, p. 832-833).

Furthermore, a motricity asymmetry (hemibody chorea and weakness of lateral control of the hemibody) can be the initial manifestation of a dysfunction in a neuro-muscular junction, such as myasthenia) (Ong et al. Singapore Med. J. 1993, vol. 34, p. 60-61).

At the present time, various techniques are used for the detection and the analysis of dyskinesia :
- A clinical quantification can be obtained upon the graduations of the severity of a dyskinesia starting from score 0 up to score 4 (Burkhard et al., Mov. Disord. 1999, vol. 14, p. 754-763). These graduations take into account a left-right asymmetry (they can be more present upon one side) and the type of dyskinesia. However, these graduations are subjective and their reproducibility depends upon the experience of the clinician.
- A quantification by videos requires well trained users. However, reproducibility between different clinicians is not adequate.
- A quantification by "accelerometry" requires the use of different "accelerometers" having various technical drawbacks that do not provide information upon muscular activity patterns associated with dyskinesia or concerning the motor behaviour during the evolution of the disease.

Furthermore, there is no monitoring of this disease following the administration of therapeutical products, such as dopaminergic agonist lévodopa or continuous administration of drugs, such as Apomorphine that requires a sub-cutaneous administration by a pump).
The flow and the concentration of these drugs require a monitoring following a clinical evaluation of the dyskinesia (Poewe et al. 1997).

Furthermore, if the detected dyskinesia has a biphasic character, the administration of apomorphine by a sub-cutaneous way is realised in bolus (Poewe et al. 1997).

It is also known that peripheral nerve stimulation by an electrical or magnetic stimulus induces modulation of electromyographic activity and muscular response. This stimulus can be administrated at different of muscular response synchronisation. However, the effects of these repetitive electrical stimuli upon kinematic and electromyographic activities with dyskinetic patients are not well known.

### Aims of the invention

The present invention aims to provide a new system, which improves the detection, the monitoring and possibly the treatment of patients suffering from dyskinesia.

Another aim of the present invention is to provide such system which do not present the drawbacks of the system of the state of the art; in particular, a system which is portable and wireless and which can be used directly by various clinicians or patients suffering from these dyskinesia.

### Summary of the invention

The present invention is related to a portable wireless electronic system for detecting, monitoring and/or treating human dyskinesia, said system comprising at least the following elements :
- a master unit,
- a measuring unit,
- a video recorder,
- a software platform,
- a (preferably detachable) memory device, and :

- possibly, a drug delivery device and batteries for maintaining the system portable.

Preferably, the measuring unit comprises sensors, more preferably inertial or muscular sensors. Inertial sensors are preferably selected from the group consisting of gyroscopes and/or accelerometers. Muscular sensors are preferably electromyographic (EMG) sensors, electroencephalographic (EEG) sensors or cortical sensors.

Preferably, these sensors are able to operate in a frequency range between 0.01 Hz and 30 Hz, preferably between 0.05 Hz and 18 Hz, more preferably between 0.5 Hz and 14 Hz.

Furthermore, the sensors may incorporate a band-pass filtering (0.3 to 25 Hz); the sensor gain can be adapted to render 1,5V at maximum angular velocity (300deg/s) .

In the system according to the invention, the master unit comprises a micro-controller, possibly a battery and a visualisation unit (such as a LCD display). Advantageously, the detachable memory device is a memory stick or a memory card.

In the system of the invention, the software platform is connected to a personal computer or a notebook, or a handheld computer device and is connected to the master unit to receive and record the set of data by the measuring unit transmitted by the master unit.

Preferably, the drug delivery device is a sub-cutaneous pump to be placed in the abdomen of the patient and able to deliver a therapeutical or prophylactic drug at a given rate, preferably automatically. Said drug being a drug able to treat or prevent diseases inducing these human dyskinesis or prevent symptoms induced by this disease.

According to a preferred embodiment of the present invention, the system according to the invention comprises a measuring unit with at least four sensors, preferably two surface electromyographic EMG sensors (to be placed over the flexor and extensor muscles) and two triaxial gyroscope sensors (to be placed over the third metacarpal and over the edge of the forearm).

According to a second embodiment of the present invention, the sensor is integrated into a textile wherein the connexion required for the sensors will be established underneath this textile.

Indeed, a textile may consist basically of an elastic shirt made on neoprene. The fabric should be elastic and with a high friction coefficient (i.e. neoprene) with respect to skin to ensure a good fitting and reliable measurements. The sensors are placed on the top of the shirt. The conductors are placed underneath the fabric in a sandwich-like approach. Sensors are clamped to the conductors through the layers of the textile substrate so that it is not possible to have an access to the conductors without dismantling the shirt. The conductors have a flat profile in order to achieve the better possible integration.

This configuration is used in order to avoid the destruction of the connexion by the patient or develop any stress upon the patient (that may induce a strangulation by the patient) that may affect the measures realised by the connexion between the master unit and the measuring unit. All connexions between the master unit, measuring unit, drug delivery device, video recorder, software platform and detachable memory device could be done by cable or preferably by means of a wireless communication network (given the high risk of using cables in psychiatric patients, who could for instance make a suicide attempt with cables).

A last aspect of the present invention is related to a method for detecting, monitoring and/or possibly treating human dyskinesis by using the portable wireless electronic system according to the invention upon a patient.

### Short description of the drawings

Fig. 1 represents schematically the system according to the invention.

Fig. 2 represents a solid model representation of the forearm.

Fig. 3 represents a biochemical parameter per segment for the biochemical model and design of the upper limb.

Fig. 4 represents pressure turn-around threshold map.

Fig. 5 represents physiological warms.

Fig. 6 represents possible positioning for tri-dimensional gyroscope present in the system according to the invention.

Fig. 7 represents tri-dimensional gyroscope sensor of the system according to the invention.

Fig. 8 represents the overall schematic representation of a preferred embodiment of the system according to the invention applied upon a human body.

Fig. 9 represents a textile carried by a patient and comprising sensors.

### Detailed description of the invention

The system according to the invention is an (preferably portable and wireless) electronic system for analysing human dyskinesis made of six components.

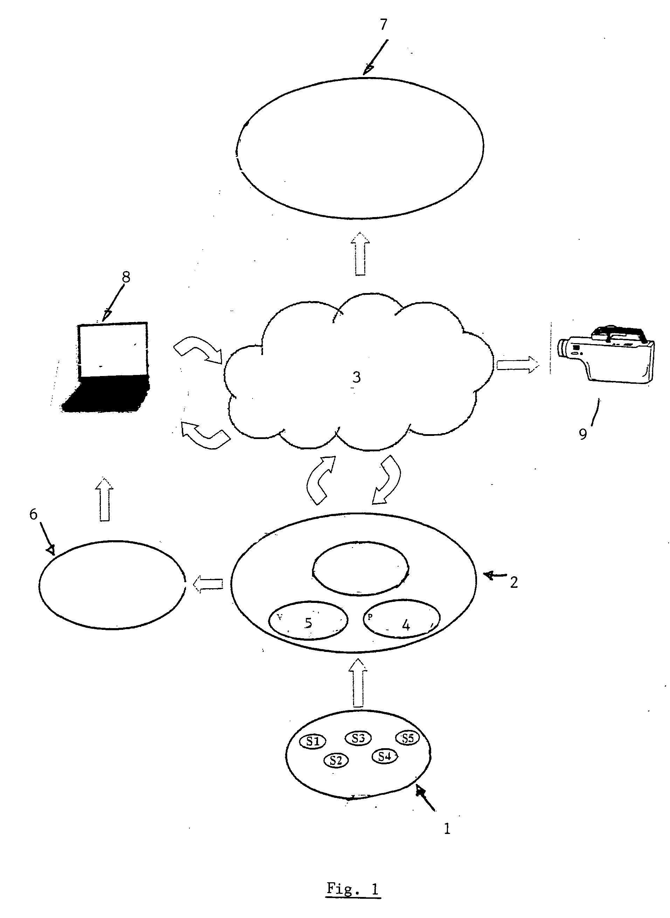

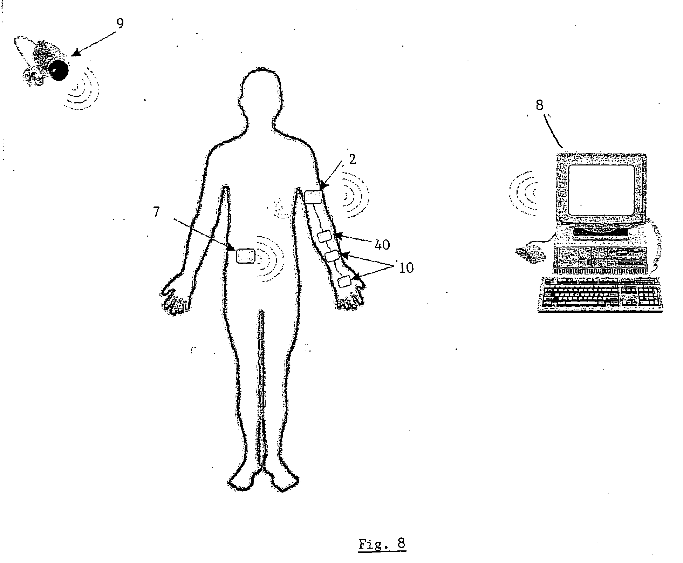

As shown in fig. 1 or fig. 8, the proposed system comprises a measuring unit (1) comprising a set of sensors. These sensors are preferably inertial sensors, such as gyroscopes (10) or accelerometers (11) able to measure all the aspects of the 3 D cinematic activity of the upper limb of a body, preferably a human body.

The other types of sensors possibly comprised in the system of the invention are electromyography (EMG) sensors (40), electro-encephalography (EEG) sensors or cortical sensors able to measure electrical activities of the upper limb muscles of the body, preferably a human body. These sensors take preferably the form of electrodes, such as surface electrodes or needle or fine-wire electrodes.

The both type of sensors above described (inertial and muscle sensors) should be able to operate in a frequency range, approximately comprised between about 0.01 Hz and about 30 Hz, preferably between about 0.05 and about 18 Hz, preferably between about 0.5 and about 14 Hz.

The system according to the invention also comprises a master unit (2) connected to the measuring unit (1) and collecting the data from each sensor of the measuring unit (1). The master unit (2) treats the collected data and performed four different tasks with the treated data :
- generates command signals in order to control the device (7) responsible for a drug delivery;
- provides the relevant information regarding a human body upon which the sensors are applied and directly available to a clinician;
- transmit the acquired data to a software platform (8) ;
- save the acquired data or processed information in a memory device (6) (such as a memory card or memory stick).

The communication between the master unit (2) and the other elements comprised in the system is implemented by cables or preferably by means of a wireless communication network (3) using (for example, the Bluetooth technology, HomeRF, the WiFi technology or the IrDA technology well known by the person skilled in the art).

The master unit (2) of the system according to the invention is an electronic board with a microcontroller (Atmega 128, Atmel Inc) connected to a LCD display (5) (o column x 2 lines character display) and a Bluetooth module. There are 8 ADC channels for each sensor. One of them is shared with the battery level meter. The resolution of each channel is up to 10 bits.

The system uses the wireless Bluetooth technology to communicate between the different units, the platform and the video-recorder. This Bluetooth technology is an open specification for short-range wireless communication between electronic devices. This system uses a Bluetooth module (Bluegiga Wrap Thor) in each unit. Medical telemetric device avoid problems with a cable. A wireless network could be established using this technology.

The master unit (2) further comprises a visualization unit (5), such as a LCD display screen, which is able to present on demand relevant information to the clinician (such as data obtained from sensors signals, details regarding the drug delivery device (7) performance or the status of the communication between the master unit (2) and the drug delivery device (7)).

In addition, the master unit (2) further contains a detachable memory device (6) (such as a memory card or a memory stick) which comprises data collected from the measuring unit (1) and data collected from the signals generated to control the device (7) responsible for the drug delivery. These data are stored for future analysis in a software platform (8), installed preferentially in a personal computer, a notebook or a handheld computer device.

A memory stick used is for instance a SanDisk 256 MB Memory Stick (SDMS-256-822). The video camera used is preferably a video Canon NTSC ZR 40. The Electromyographic sensor used can be an active Electromyographic sensor or amplifier, like the 2.1 Delsys surface electrode.

The base unit preferably also comprises a Bluetooth mode to establish a wireless link with the master unit. This system is powered by lithium ion batteries (CGA-7/102F, 3.7V, 900 MaH, Matsushita Inc.).

The master unit (2) is also connected to an external digital video recorder (9), preferably via a wireless communication network and is responsible for the generation of synchronization signals in order to synchronize the acquisition of the digital video recorder (9) and the measuring unit (1).

The power supply of the master unit (2) and its components (1,9,7,8) is provided by batteries (4) in order to maintain the system portable and independent.

The proposed system also comprises a software platform (8) which is optionally connected to the master unit (2) for receiving and recording the set of data collected by the measuring unit (1) and transmitted by the master unit (2). The software platform collects, treats and presents the data result under an appropriate form to a clinician, preferably upon the visualization unit (5), such as the LCD display.

In other words, both the master unit (2) and the software platform (8) are able to perform the analysis of the data acquired by the measuring unit (1). However, it is only the master unit (2) which is able to generate signals to control the drug delivery device (7).

In addition, the software platform (8) is a configuration tool, where the clinician could set up different parameters of the system according to the invention, such as the sampling frequency of the acquisition system or the characteristics of the signal used to obtain a synchronization of the video recorder system (9) with the acquisition system (measuring unit).
This configuration parameters are preferably transmitted to the master unit (2).

In the system according to the invention, the drug delivery device (7) is responsible for a delivery of a drug to the body, preferably a human body. Such device is for example a subcutaneous pump placed in the abdomen which delivers a drug at a given flow rate.

The video recorder (9) of the system according to the invention allows the assessment of the general neurological status of the patient, in particular the presence or the absence of involuntary movements in lower limbs and the awakeness of the patient during diagnosis and/or treatment (by the sensors of the measuring unit (1) and during drug delivery).

### Position of the sensors upon the human body

### Biomechanical model and design of the upper-limb

A biomechanical model of the upper arm is used to monitor and analyse the movements of a human arm. The biomechanical model built takes into account the Leva (Leva, 1996) corrections and the biomechanical tables of Zatsiorsky and Seluyanov (1990), Zatsiorsky VM, Seluyanov V. The mass and inertia characteristics of main segments of human body. Proceedings of Biomechanics VII-A. Morecki A, Fidelus K (Ed) 1981; Leva P. Adjustments to Zatsiorsky-Seluyanovs's segment inertia parameters. J. Biomech 1996; 9: 1223-1230; Denavit J, Hartenberg RS. A kinematic notation for lower-pair mechanisms based on matrices. J. App. Mech. 1955; 77: 215-221. These tables are the most widely accepted within the field of biomechanics dynamic analysis; in particular, in sports and medical biomechanics. Leva adjustments are used to define accurately the anthropometric measurements required to obtain inertial parameters from Zatsiorsky tables. From these tables a forearm solid rigid model is built. This model has been parametrised using the Denavit-Hartenber approach (1995).(references completes, svp).

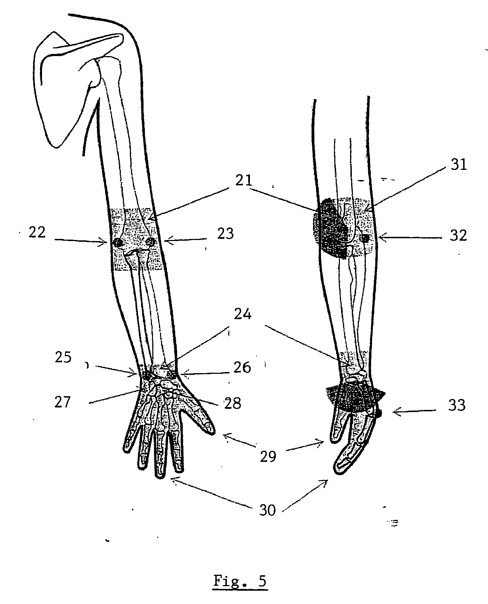

For a Denavit-Hartenberg parametrisation, four rigid segments are defined, in order to obtain analysis of all the recorded degrees of freedom. Each segment is responsible for a degree of freedom :
1. Elbow flexion-extension
2. Pronation-supination
3. Wrist flexion-extension
4. Wrist deviation
Two of these segments are virtual (with no mass and no length) and each segment has attached its own reference system (plus a coordinate frame for all of them) (see fig. 5 representing the coordinate frame defined and the degree of freedom represented for each system).

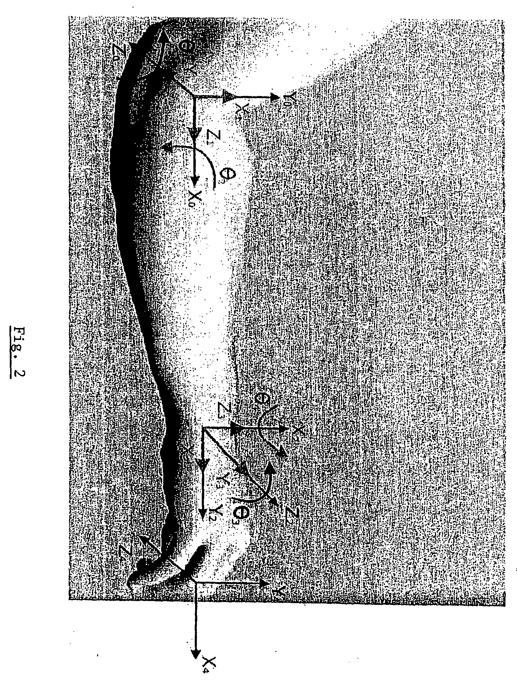

The Denavit-Hartenberg parameters are also present in the following table 1 in reference to figure 2.

For rotary elements, the parameter θ determines the position of the joint. Therefore, in this table, it is indicated the relationship between the parameter and the physiological measured angle represented by the letter β.

**Table 1**

| Segment | D | A | θ | A |
|---|---|---|---|---|
| 1- Elbow F/E | 0 | 0 | β+π/2 | π/2 |
| 2- Pronation | F_{L} | 0 | β | π/2 |
| 3-Wrist F/E | 0 | 0 | β+π/2 | π/2 |
| 4-Wrist Dev | 0 | H_{L} | β | π/2 |

| | | | | |
|---|---|---|---|---|
| (F_{L} means forearm length and H_{L} means hand length. α = Angle between Zᵢ to Zᵢ₊₁ measured about Xᵢ a = Distance from Zᵢ and Zᵢ₊₁ measured along Xᵢ d = Distance from Xᵢ₋₁ and Xᵢ measured along Zᵢ θ = Angle between Xᵢ₋₁ and Xᵢ measured about Zᵢ | | | | |

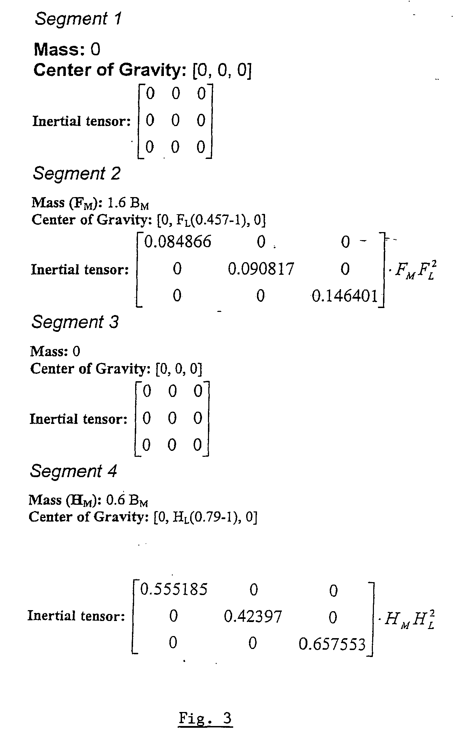

Furthermore, biomechanical parameters per segment have to be obtained from Leva (1996). Segment 1 and segment 3 are virtual. They are only defined to manage the degree of freedom of elbow flexion-extension and wrist flexion-extension respectively. But when these segments are moved, they move the masses of the "real" segments. All inertial and mass parameters per segment are defined below. The following symbols of fig. 3 are used :
- B_{M} : body mass
- F_{L} : forearm length
- F_{M} : forearm mass
- H_{L} : hand length
- H_{M} : hand mass

### Biomechanial constrains

The sensor devices allocated on the upper limb jointly with the "sleeve" containing them comply with the biomechanical requirements for general upper limb textile. The following data represent the most important requirements related to pressure and physiological warns.

For pressure tolerance threshold map of the upper-limb for textile design (it is necessary to take care of maximum pressure which is tolerated).
15 = Low tolerance area (average near 450kPa)
16 = Middle tolerance area comprised between 450kPa and 950kPa)
17 = High tolerance area (average near 950kPa)

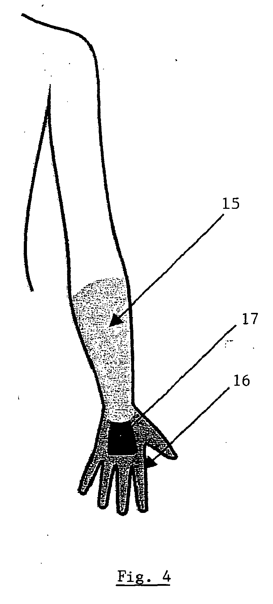

The map of fig. 4 gives maximum values of this pressure between the hand and the forearm of a human patient that needs to be avoided, in order to keep a comfortable design of the system.

The fig. 5 represents the physiological warns. These areas cannot be used to allocate sensors:

**Physiological warns**

| | | | |
|---|---|---|---|
| 21. | Elbow movement area | 27. | Guyon tunnel |
| 22. | Medial epycondile | 28. | Carpal tunnel |
| 23. | Lateral epycondile | 29. | Thumb movement area |
| 24. | Wrist movement area | 30. | Fingers movement area |
| 25. | Radial styloid process | 31. | Cubital tunnel |
| 26. | Ulnar styloid process | 32. | Olecranon process |
| | | 33. | Metacarpal heads |

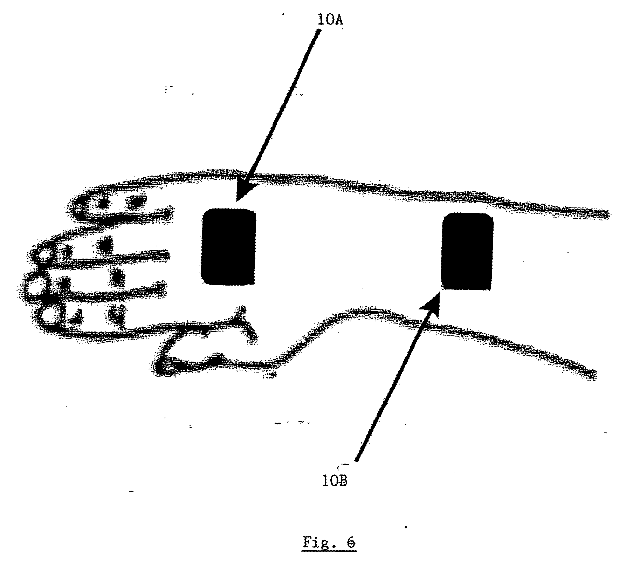

The following measurements are obtained with the various sensors of the measuring unit :
a) for gyroscopes (10) : they are used to measure the speed of the three-dimensions in space. These speed values are collected and formed a vector which is the addition of the various absolute speeds.
b) Cutaneous electromyographic sensors : they are used for collecting muscle activities (agonist, antagonist and synergic muscle activities).
c) Electromyographic needle electrodes : they are used to measure and collect motor unit activities as well as denervation signs such as fibrillations, detect and collect data related to the muscular activities.
The enclosed fig. 6 represents a possible positioning for three-dimensional gyroscopes of the measuring unit.

However, this measuring unit can also comprise other sensors, such as two surface EMG sensors (11) placed over the flexo and extensor muscles and two other sensors which are the two triaxial gyroscope sensors, a first gyroscope (10A) placed over the third metacarpal placed while the gyroscope (10B) is placed over the edge of the forearm. The measurement of the wrist flexo/extension angular speed is therefore obtained by the subtraction obtained from gyroscope (10A) from gyroscope (10B).

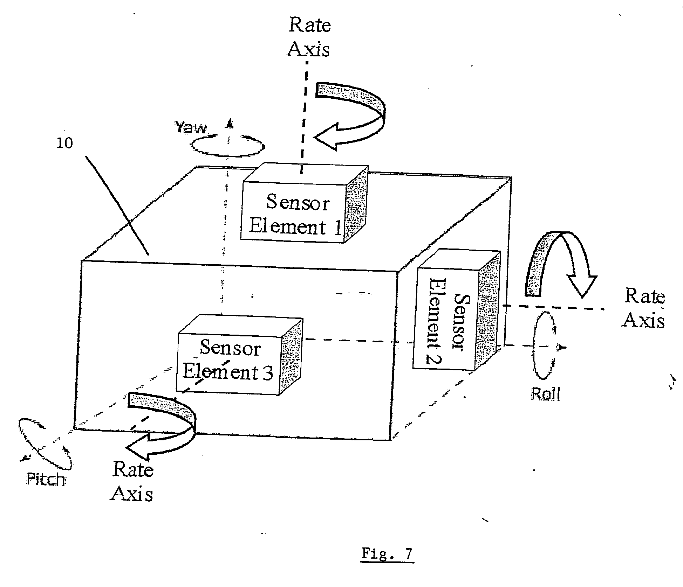

As shown in the fig. 7, the three-dimensional gyroscopes sensors (10) according to the invention, measure absolute angular velocity.

Therefore, two of these gyroscopes are required to get a tremor contribution of each joint.

The sensor elements of the gyroscope sensors will just measure angular velocity in one axis.

A tri-dimensional measurement of the movements is achieved based upon the use of three elements sensor mechanically placed orthogonally between them (see fig. 5). The system may also include EMG sensors (40) as represented on figure 8.

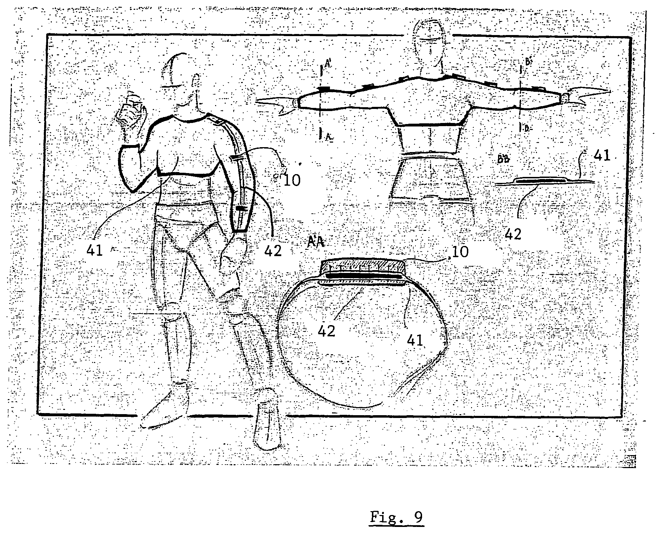

As represented in the fig. 9, the patient will wire a textile, such as a neoprene shirt (41) with sensors. The connections (42) required for the sensors (40) are therefore incorporated into the textile element. Preferably, the sensors (41) are placed on top of the shirt while the conductors (42) are placed underneath the fabrics in a sandwich-like element.

Sensors are clamped to the conductors through the layers of the textile element. Therefore, it is not possible to have an access to the conductors without dismantling the shirt (41).

Preferably, the conductors (42) have a flat profile in order to achieve the better possible integration.

This type of textile is preferably used to avoid stress for the person during the diagnosis, the monitoring and/or the therapy of dyskinesia.

## Claims

1. An electronic system for the diagnosis, the monitoring and/or the treatment of dyskinesia comprising :
- a measuring unit (1) with sensors to be applied upon a human body patient,
- a Master Unit (2),
- a video recorder (9),
- a software platform (8),
- a memory device (6),
- and a drug delivery device (7) to be applied upon the human body patient.

2. The system according to claim 1, wherein the measuring unit (1) comprises inertial and muscle sensors.

3. The system according to claim 2, wherein the inertial sensors are selected from the group consisting of gyroscopes (10) and accelerometers (11).

4. The system according to claim 2, wherein the muscle sensors are electromyographic sensors (40), electro-encephalographic sensors or cortical sensors.

5. The system according to the claims 2 to 4, wherein the sensors are able to operate in a frequency range between 0.05 Hz and 30 Hz, preferably between 0.05 Hz and 18 Hz and more preferably between 0.05 Hz and 14 Hz.

6. The system according to any of the preceding claims, wherein the Master Unit comprises a micro-controller (3), a visualisation unit (5) such as a LCD display and possibly a battery (4).

7. The system according to any of the preceding claims, wherein the memory device (6) is a detachable memory stick or a detachable memory card.

8. The system according to any of the preceding claims, wherein the software platform is integrated in a personal computer, a notebook or a handheld computer device.

9. The system according to any of the preceding claims, wherein the software platform (8) is connected to the master unit (2) to receive and record the set of data by the measuring unit (1) and transmitted by the master unit (2).

10. The system according to any of the preceding claims, wherein the drug delivery device (7) is a subcutaneous pump placed in the abdomen of the human body patient and able to deliver a drug at a given rate.

11. The system according to any of the preceding claims, wherein the measuring unit (1) comprises four sensors, two surfaces electromyographic sensors (12) and two tridimensional (triaxial) gyroscopes sensors (10).

12. The system according to any of the preceding claims, wherein the sensors are integrated into a textile (41) carried by the human patient suffering from dyskinesia.

13. The system of claim 12, wherein the textile is neoprene.
